# EUROPEAN PATENT SPECIFICATION

(11) **EP 0 597 969 B1**
(45) Date of publication and mention of the grant of the patent: **10.11.1999**
(21) Application number: 92916915.9
(22) Date of filing: 07.08.1992
(51) Int. Cl.: C12N 1/20

(54) **PROCESS FOR THE PRODUCTION OF THERMOPHILIC MICROORGANISMS IN HIGH YIELD**
HERSTELLUNGSVERFAHREN FÜR THERMOPHILE MIKROORGANISMEN MIT HOHER AUSBEUTE
PROCEDE DE PRODUCTION DE MICROORGANISMES A GRAND RENDEMENT

(30) Priority: 09.08.1991 GB 9117209
(43) Date of publication of application: 25.05.1994
(73) Proprietor: MICROBIOLOGICAL RESEARCH AUTHORITY, Salisbury, Wiltshire SP4 0JG (GB)
(72) Inventor: RAVEN, Neil D. H., Public Health Lab. Serv. Board, Salisbury, Wiltshire SP4 0JG (GB); COSSAR, John D., Public Health Lab. Serv. Board, Salisbury, Wiltshire SP4 0JG (GB); LADWA, Narendra M., Public Health Lab. Serv. Board, Salisbury, Wiltshire SP4 0JG (GB); SHARP, Richard J., Public Health Lab. Serv. Board, Salisbury, Wiltshire SP4 0JG (GB)
(74) Representative: Schlich, George William
(86) International application number: GB9201470
(87) International publication number: WO9303136

(56) References cited:
- THE JOURNAL OF BIOLOGICAL CHEMISTRY. vol. 264, no. 9, 25 March 1989, BETHESDA, US. pages 5070 - 5079 F.O. BRYANT & M.W.W. ADAMS. 'Characterization of hydrogenase from the hyperthermophilic archaebacterium, Pyrococcus furiosus.' cited in the application
- APPLIED BIOCHEMISTRY AND BIOTECHNOLOGY. vol. 18, 1988, CLIFTON, NEW JERSEY. pages 53 - 73 A.K. PARAMESWARAN ET AL. 'Engineering considerations for growth of bacteria at temperatures around 100 C.' cited in the application
- ANNALS OF THE NEW YORK ACADEMY OF SCIENCES. vol. 589, 20 May 1990, US. pages 301 - 314 I.I. BLUMENTALS ET AL. 'The hyperthermophilic archaebacterium, Pyrococcus furiosus: development of culturing protocols, perspectives on scaleup, and potential applications.' cited in the application
- ARCHIVES OF MICROBIOLOGY. vol. 149, no. 2, 1987, SPRINGER, BERLIN. pages 95 - 101 R. HUBER ET AL. 'Pyrobaculum gen. nov., a new genus of neutrophilic, rod-shaped archaebacteria from continental solfataras growing optimally at 100 C.' cited in the application
- PATENT ABSTRACTS OF JAPAN vol. 12, no. 209 (C-504)(3056) 15 June 1988

## Description

This invention relates to a process for the production of thermophilic archaea in high yield.

A remarkable characteristic of the thermophilic archaea is their ability to grow at extremely high temperatures. In nature, these organisms grow in geothermal areas such as terrestrial solfataric springs and mud holes and in marine hydrothermal areas, as well as outflows from geothermal powerplants.

Many workers have realised the potential applications of both thermophilic microorganisms and their thermophilic proteins in industrial processes. Large quantities of microorganisms are consequently required for use in these processes and high yields and reproducible conditions are therefore required. However, the nature of their growth requirements and the sometimes extreme conditions associated therewith present many difficulties in devising procedures to achieve these objectives.

The provision of a process for the production of thermophilic archaea on a large scale would therefore be useful in order to study the thermophilic proteins produced and their potential industrial applications.

Many of these organisms obligately evolve hydrogen sulphide as an end product of their metabolism by the reduction of elemental sulphur or some of its compounds (eg sulphite and thiosulphate). Others, however, are capable of evolving hydrogen as an alternative in the absence of elemental sulphur.

As an example of the latter *Pyrococcus* is, a genus of thermophilic bacteria belonging to the archaeal domain and first described by Fiala and Stetter in 1986 (Arch. Microbiol, 145: 56-61). *P furiosus* was first isolated from geothermally heated marine sediments and is able to grow in a temperature range between 70 and 103°C and at pH 5-9 and 0.5-5.0% NaCl. Its optimum growth temperature is 100°C (Fiala and Stetter, supra).

*P furiosus* is a strictly anaerobic heterotroph and grows by a fermentative-type metabolism. The cells are spherical and Gram-negative, being about 0.8 to 2.5µm in width and possess monopolar polytrichous flagella and a granum-like body visible under light microscopy. Both simple and complex carbohydrates can be utilised, with the production of CO₂, H₂ and acetate in the absence of elemental sulphur.

In the past, attempts at devising procedures for culturing thermophilic archaea in high yields have resulted in yields no greater than 3 x 10⁸ cells/ml (even in the presence of elemental sulphur). In addition, attempts at overcoming problems caused by specific growth requirements or production of hazardous waste products have hindered the production of higher cell densities. H₂S corrosion when sulphur is present in the medium has necessitated the use of expensive H₂S-resistant materials for the construction of culture apparatus.

Parameswaran et al, Appl. Biochem. Biotechnol. 18. 53-73 (1988) discloses that *P. furiosus* grows better in the presence of elemental sulphur (reducing the inhibitory effect of H₂ on cell growth), but a consequence is that reactor materials are likely to be corroded by the resultant large volume of hydrogen sulfide produced. The reference also discloses that for culture of *P. brocki* lowering H₂ partial pressure may reduce H₂S associated problems, but only at the expense of lower cell yields. Modified conventional fermentors, gas lift systems and membrane bioreactors are disclosed as possible bioreactors.

Bryant and Adams (J. Biol. Chem. 1989, vol 264 No 9) disclose a procedure for batch culturing *P furiosus* in sulphur-free media, sparged with Ar to relieve inhibition by H₂. The yields obtained from this procedure were however extremely variable, ranging between 450 and 1100g (1-3g/litre) wet weight of cells and cell density of up to 1 x 10⁸ ml⁻¹ from a 400-litre fermenter.

Huber et al (Arch. Microbiol 1987 149:95-101) discloses batch culture of *Pyrobaculum islandicum* (an obligately H₂S - evolving thermophilic archaea). Cell densities of up to 3 x 10⁸ml⁻¹ were obtained in stirred N₂ sparged cultures in a 3001 enamel-protected fermenter using thiosulphate as electron acceptor.

It is thus a disadvantage of the prior art that cultures of archaea grown on medium free of elemental sulphur can only achieve cell densities of up to 3 x 10⁸ ml⁻¹ even in batch culture.

It is an object of the present invention to provide a process for the production of thermophilic archaea which provides higher cell yields. It has now surprisingly been found that high growth yields of thermophilic archaea in excess of any achieved by prior art processes can be obtained, while operating under steady state conditions.

Thus, according to the present invention, there is provided a process for producing thermophilic archaea in medium essentially free of elemental sulphur and wherein an inert gas is continuously introduced into the medium so as to remove hydrogen and/or hydrogen sulphide evolved by the archaea characterised in that said archaea are grown continuously in the presence of a nutrient medium containing assimilable sources of carbon and nitrogen and any necessary complex nutrients required for cell growth, and in that the inert gas is continuously introduced into the culture medium at a rate of between 0.1 and 1 vv⁻¹min⁻¹.

In a preferred operating mode for the process of the invention, the temperature of the culture is maintained at between 70 and 110°C. For *P. furiosus,* the preferred temperature range is 70 to 103°C, more preferably 80 and 100°C, and most preferably it is maintained at 90°C.

For *P. islandicum* the preferred temperature range is 74°C to 102°C, more preferably 90°C to 100°C and most preferably around 95°C.

It is likewise a preferred feature of the process of the invention that the culture medium in the reactor is maintained at a concentration of ionised salts which effectively mimics the ambient conditions in the natural environment of said thermophilic archaea.

Thus it is preferred that a suitable concentration of an acceptable salt is maintained in the medium.

Examples of said acceptable salts are the chlorides and bromides of sodium, potassium, magnesium and calcium.

For marine archaea eg *P. furiosus,* it is most preferred that the medium comprises sodium chloride.

It is further preferred that the total salt concentration in the medium is maintained at between 0.1 and 1M, most preferably at 0.5M.

For terrestrial archaea eg *Pyrobaculum islandicum* it is preferred that the total salt concentration is maintained between 0.001M and 0.1M, most preferably 0.02M.

It is preferred that the inert gas continuously introduced is selected from nitrogen, argon, helium, and sulphur hexafluoride, and it is most preferred that it is nitrogen. The inert gas according to the invention is continuously introduced into the culture medium at a rate of between 0.1 and 1 vv⁻¹min⁻¹. Preferably, it is introduced at 0.5 vv⁻¹min⁻¹. For *P. islandicum* the preferred rate is around 0.2 vv⁻¹min⁻¹.

The reactor most preferably used in the process of the invention is a gas-lift fermenter.

The invention will now be described by way of examples with particular reference to the accompanying drawings in which:-
Fig. 1 is a schematic drawing of a continuous culture reactor vessel and ancillary apparatus;
Fig. 2 is a bar chart showing relative cell densities achieved with different sparging gases under equivalent conditions;
Fig. 3 shows the results of continuous culture of *Pyrococcus furiosus* in the absence of elemental sulphur (90°C, pH7.0, dilution rate 0.2h⁻¹) and the time course of the variation in cell density of a 51 culture with argon flow rates from 60ml min⁻¹ to 600ml min⁻¹ (0.012 - 0.12v v⁻¹ min⁻¹);
Fig. 4 shows the results of continuous culture of *Pyrococcus furiosus* in the absence of elemental sulphur (90°C, pH7.0. dilution rate 0.2h⁻¹) and the effect of argon flow rate upon equilibrium cell density (bold line - 21 culture, fine line - 51 culture);
Fig. 5 shows the results of continuous culture of *Pyrococcus furiosus* in the absence of elemental sulphur (90°C, pH7.0, dilution rate 0.2h⁻¹ and the equilibrium cell densities of *Pyrococcus furiosus* with four inert gases, argon (Ar), helium (He), nitrogen (N₂) and sulphur hexafluoride (SF₆) at a flow rate of 0.3v v⁻¹ min⁻¹;
Fig. 6 shows the results of continuous culture of *Pyrococcus furiosus* in the absence of elemental sulphur (90°C, pH7.0, dilution rate 0.2h⁻¹) and the effect of nitrogen flow rate upon eqilibrium cell density;
Fig. 7 shows the results of continuous culture of *Pyrococcus furiosus* in the absence of elemental sulphur (90°C, pH7.0, nitrogen flow rate 0.5v v⁻¹ min⁻¹). Effect of dilution rate upon equilibrium cell density; and
Fig. 8 shows the results of continuous culture of *Pyrococcus furiosus* in the absence of elemental sulphur (90°C, pH7.0, nitrogen flow rate 0.5v v⁻¹ min⁻¹) and the effect of dilution rate upon biomass production (bold line - biomass per litre of culture eluate, fine line biomass production per hour per litre of culture volume (21)).

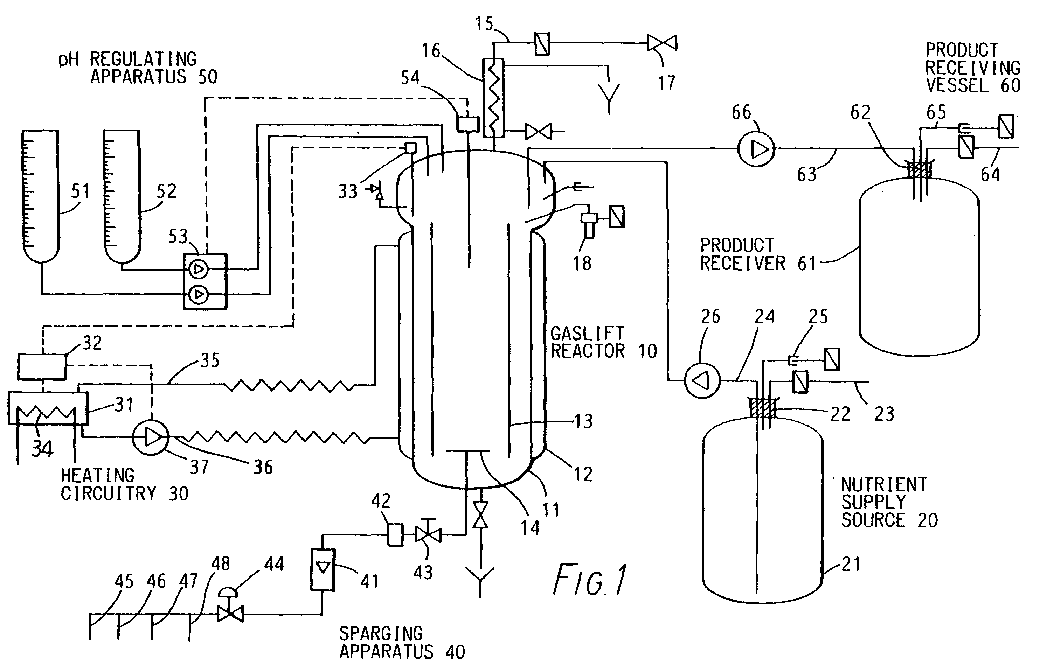

Figure 1 is a schematic drawing of a continuous culture reactor vessel and ancillary apparatus consisting of gas lift reactor 10. nutrient supply source 20, heating circuitry 30, sparging apparatus 40, pH regulating apparatus 50 and product receiving vessel 60.

The gas lift reactor comprises a cylindrical glass fermentation vessel 11 surrounded by a heating jacket 12. An annular draft tube 13 is located concentrically within the vessel for a purpose to be described below. At the lower end of vessel is located a sintered glass sparger 14, and at the upper end is located a gas outlet 15 provided with a condenser 16 and exhaust 17. A sample valve 18 is also provided at the upper end of vessel

Nutrient supply source 11 comprises a cylindrical vessel 21 having a narrow opening at one end and which is sealed with a removable bung 22. Said bung is perforated by three holes through which pass an inlet pipe 23, an outlet pipe 24 and a gas balance line 25. The outlet pipe is connected through a pump 26 to the top end of vessel.

The heating circuitry comprises a heater 31 and a temperature control 32 connected in series to a temperature sensor 33 which enters the vessel at the upper end. The heater comprises a heating element 34 and inlet and outlet pipes 35 and 36 connected to the heating jacket 12. The outlet pipe 36 is provided with a pump 37.

The sparging apparatus comprises a flow meter 41, a filter 42 and a needle valve 43, linked in series by pipework to a sintered glass sparger at one end and a regulating valve 44 at the other. Gas sources 45 to 48 are linked by pipework to the regulating valve.

pH regulating apparatus 50 comprises acid and alkali reservoirs 51 and 52, connected by pipework in parallel to the upper end of the vessel. Said pipework runs through a pH control 6 which is linked to a pH sensor at the upper end of the vessel.

Product receiver 60 comprises a cylindrical vessel 61 having a narrow opening at the upper end sealed with a removable bung 62 provided with three holes through which pass an inlet pipe 63, an outlet pipe 64 and a gas balance line 65. The inlet pipe is connected to the upper end of the vessel and is provided with a pump 66.

### Example 1

*Pyrococcus furiosus* strain Vc 1 (Fiala and Stetter 1986) was obtained from the Deutsch Sammlung von Mikroorganismen und Zellkulturen GmbH as DSM 3638.

### Growth Conditions

All growth experiments were performed under anaerobic conditions at 90°C and pH 7.0.

A modified SME medium (original SME description see Stetter et al (1983) System. Appl. Microbiol) was prepared containing the following:-

| | |
|---|---|
| Bacto peptone | 5g l⁻¹ |
| Yeast extract | 1g l⁻¹ |
| Sodium chloride | 28g l⁻¹ |
| Magnesium salts | 10ml l⁻¹ |

| | |
|---|---|
| Solution A | 1ml l⁻¹ |
| Solution B | 1ml l⁻¹ |
| Solution C | 1ml l⁻¹ |
| Resazurin (1mg ml¹) | 1ml l⁻¹ |
| Vitamin Solution | 0.5ml l⁻¹ |
| Cysteine HCL | 0.5g l⁻¹ |

| Magnesium salts | gl⁻¹ |
|---|---|
| Magnesium sulphate 7H₂O | 180 |
| Magnesium chloride 6H₂O | 140 |

| Solution A | |
|---|---|
| | g l⁻¹ |
| Trisodium citrate | 4 |
| Manganese (II) Sulphate 4H₂O | 9 |
| Iron (II) Ammonium Sulphate 6H₂O | 10 |
| Zinc Sulphate 7H₂O | 2.5 |
| Copper (II) Sulphate 5H₂O | 0.15 |
| Potassium Aluminium Sulphate 12H₂O | 0.3 |
| Cobalt (II) Chloride 6H₂O | 0.3 |
| Nickel (II) Chloride | 2.5 |

| Solution B | |
|---|---|
| | g l⁻¹ |
| Calcium Chloride 2H₂O | 56 |
| Potassium Chloride | 16 |
| Strontium Chloride | 4 |
| Sodium Bromide | 25 |
| Potassium Iodide | 10 |

| Solution C | |
|---|---|
| | g l⁻¹ |
| Dipotassium Hydrogen Phosphate | 500 |
| Sodium Tungstate 2H₂O | 3.3 |
| Boric Acid | 7.5 |
| Sodium Molybdate 2H₂O | 0.15 |
| Sodium Selenite 5H₂O | 0.005 |

| Vitamin Solution (modified from Balch et al) | |
|---|---|
| | mg l⁻¹ |
| Biotin | 40 |
| Folic Acid | 40 |
| Pyridoxine-HCL | 200 |
| Thiamine-HCL | 100 |
| Riboflavine | 100 |
| Nicotinic Acid | 100 |
| DL-Calcium Pantothenate | 100 |
| Cyanocobalamin | 2 |
| Lipoic Acid | 100 |

The stock solutions A B and C were prepared as 1000 x concentrated solutions and the vitamin solution was prepared at 2000x concentration to be used. The vitamin solution was prepared as a 50% v/v ethanol/water mix. The ethanol soluble components were first dissolved in ethanol and the water soluble components dissolved in water and the solutions mixed. In the event of precipitation, the solution was shaken before use.

The components of the medium described above were mixed in the following order when the medium was prepared:
(1) Sodium Chloride
(2) Magnesium Salts
(3) Solution A
(4) Solution B
(5) Solution C
(6) Resazurin
(7) Vitamin Solution
(8) Bacto Peptone
(9) Yeast Extract
(10) Cysteine-HCL
(11) Sodium Hydroxide

Referring to Figure 1 the medium was sterilised and transferred to vessel 21 by means of inlet pipe 23. The medium in vessel 21 was maintained under anaerobic conditions and unless otherwise stated, anaerobic conditions were maintained throughout the process.

Media was transferred through outlet pipe 24 to the reactor vessel 11 by means of pump 25 at a rate of 400ml/hr until a volume of 2 litres had been reached.

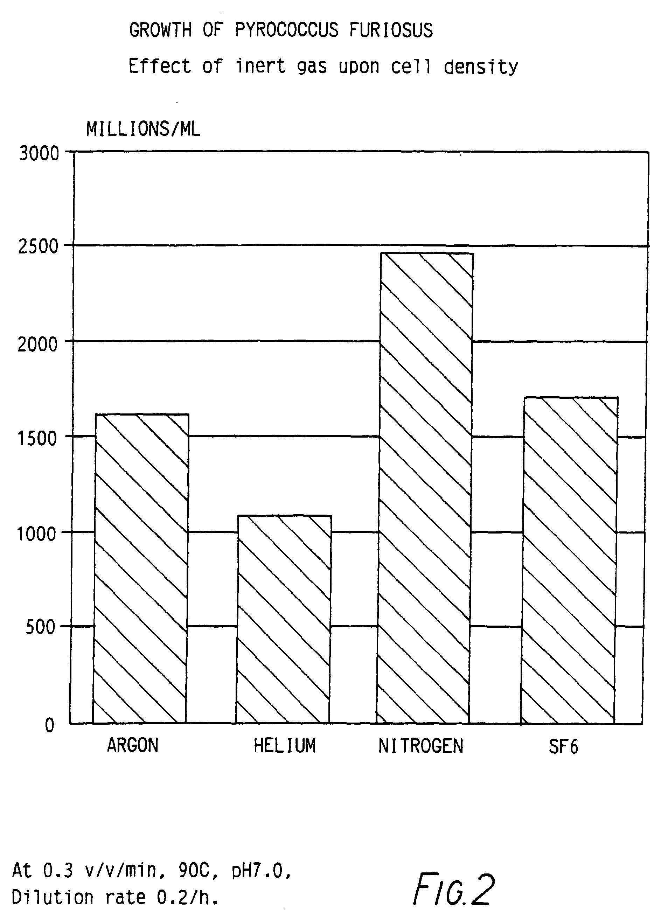

The heating circuitry 30 was used to bring the temperature of the medium in vessel 11 up to between 90-100°C. The temperature was maintained at this level by the temperature control 32. The annular draft tube 13 induces a toroidal circulation of fluid within the vessel 11. Thus bubbles generated by the sparger rise within the riser and gas disengages at the upper end of the vessel. Fermentation medium then returns to the bottom of the vessel flowing downwards in the annular space between the draft tube and the vessel walls. The sparging apparatus was then used to flush the reactor vessel 11 in order to ensure that no traces of oxygen were present, and then a constant flow rate of 0.5 vv⁻¹min⁻¹ was established. The gas entered the reactor via the sintered glass sparger 14. The sparging gas used initially was N₂. However, He, Ar, or SF₆ could also be used for this purpose, or a mixture of all four. When the reactor was run at above 95°C, the pressure in the reactor was adjusted to greater than atmospheric pressure to prevent water loss. A comparison of the efficiency of all four sparging gases was performed and the results are shown in Figure 2. As will be understood by those skilled in the art, the use of a "gas-lift" reactor of the type described herein facilitates the removal of unwanted gases from the culture whilst providing the necessary mixing which the culture requires for optimum growth. As the thermophilic archaea are more than usually susceptible to death by shearing forces caused by stirring, this kind of mixing is especially suitable when high cell densities are required.

The medium in vessel 11 was inoculated with a culture of *P furiosus* strain Vcl obtained from the Deutsche Sammlung Von Microorganismen und Zellkulturen GmbH as DSM 3638 and grown under anaerobic conditions. The rate of flow of the sparging gas was adjusted using the regulating valve until a gas flow of 0.5 vv⁻¹min⁻¹ was reached. Cell densities in the reactor were measured by extracting samples from vessel 11 using the sample valve 18.

As the purpose of this process was to achieve the highest cell density possible, all nutrients in the medium were provided in excess in order to avoid any limitation on growth rate. As will be understood by those skilled in the use of continuous culture systems, when no limitation on cell growth is imposed by essential nutrient starvation, the dilution rate in a system can be increased to a point where a maximum cell density and a minimum cell doubling time is achieved. Above this dilution rate cell density decreases due to bacteria being washed from the culture. It was found that cell densities in excess of 3 x 10⁹ cells ml⁻¹ (=4g wet/wt l⁻¹) could be maintained indefinitely at a dilution rate of 0.2 hr⁻¹ with a nitrogen flow rate of 0.5 vv⁻¹min⁻¹.

### RESULTS

### Argon as Inert Sparging Gas

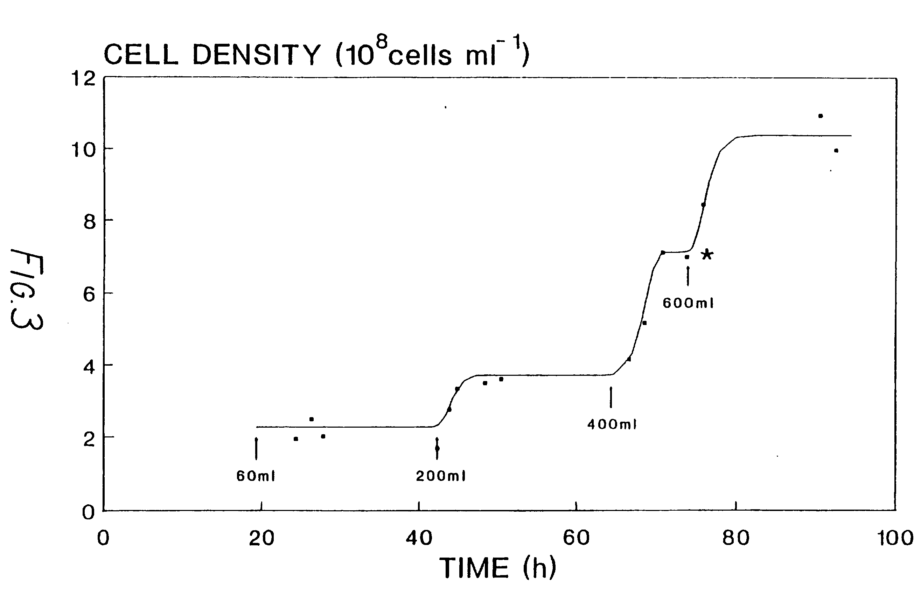

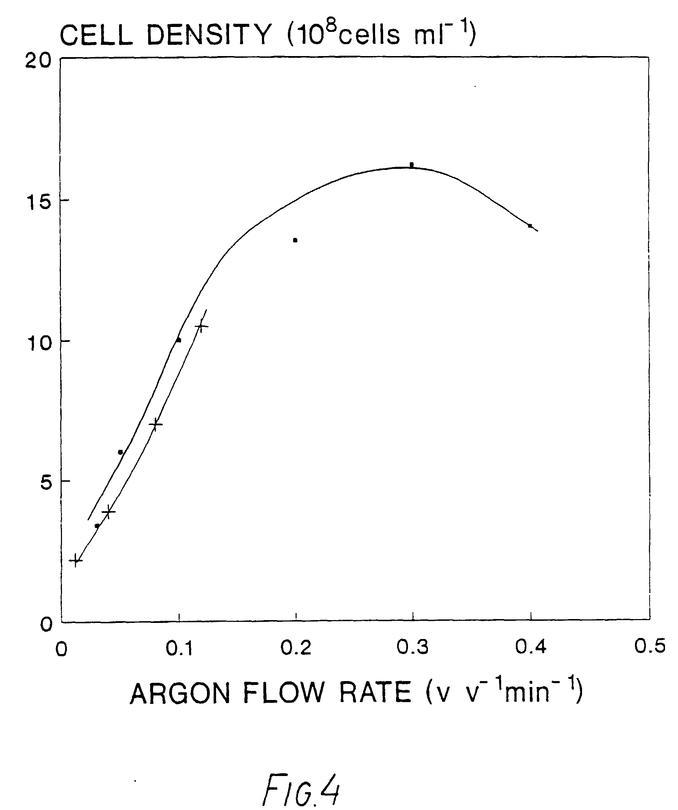

Initial experiments on continuous culture of *Pyrococcus furiosus* were performed in a bioreactor with a working volume of 51 at a dilution rate of 0.2h⁻¹ and argon as the inert sparging gas. Inhibition of growth by hydrogen produced during fermentation had been previously reported to be completely relieved by sparging with argon at 0.02 v v⁻¹ min⁻¹ (Bryant and Adams 1989). In the experimental system described, however, cell densities increased with argon flow throughout the range tested; 0.012 - 0.12 v v⁻¹ min⁻¹ (Fig. 3). Higher gas flow rates were tested, therefore, at the same dilution rate in a 21 bioreactor of the same design. Steady-state cell densities increased to 1.6 ± 0.1 x 10⁹ml⁻¹ at 0.3 v v⁻¹ min⁻¹, above which level, however, foaming of the medium increased and the equilibrium cell density declined (Fig. 4).

### Effect of Alternate Inert Sparging Gases

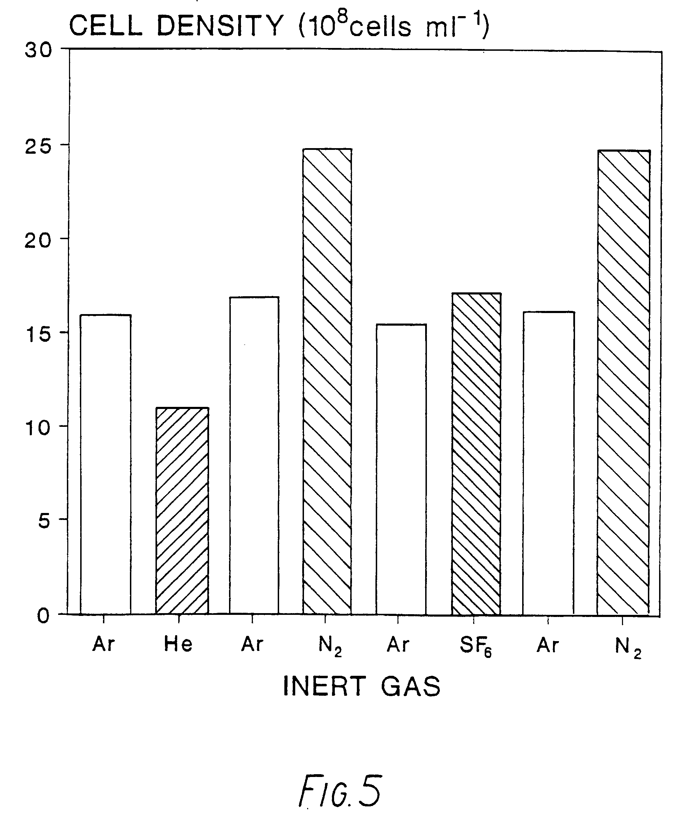

To evaluate whether argon was the optimal inert gas for the system, three other inert gases, helium, nitrogen and sulphur hexafluoride were tested. All experiments were performed under equivalent conditions with a gas flow rate of 0.3v v⁻¹ min⁻¹ and, as previously, a dilution rate of 0.2h⁻¹. The cell density with argon was determined before and after each new gas was supplied. No significant change in this cell density was observed indicating that equivalent culture conditions were present throughout the experiments. Nitrogen was found to support the highest cell densities under these conditions (2.5 ± 0.1 x 10⁹ml⁻¹) and to be significantly better than either argon (1.6 ± 0.1 x 10⁹ cells ml⁻¹). These gases in turn supported significantly higher cell densities than helium (1.1 ± 0.1 x 10⁹ cells ml⁻¹); (Fig. 5).

### Optimisation of Nitrogen Sparging Rate

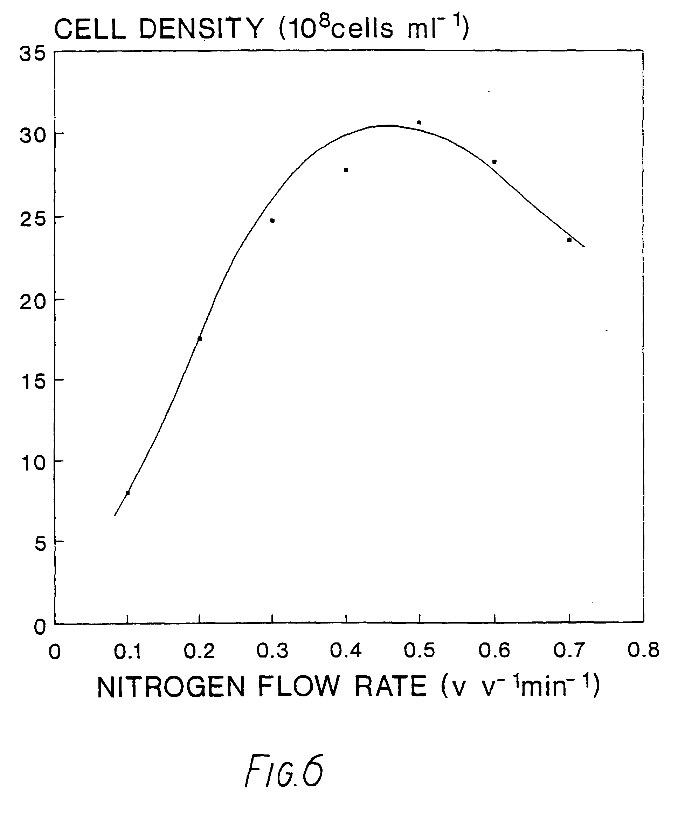

Nitrogen flow rates from 0.1 - 0.7v v⁻¹ min⁻¹ (in 0.1v v⁻¹ min⁻¹ intervals) were tested sequentially to determine the optimum sparging rate. Cell densities were found to increase with nitrogen flow up to 0.5v v⁻¹ min⁻¹, where 3.1 ± 0.1 x 10⁹ cells ml⁻¹ were maintainable at a dilution rate of 0.2h⁻¹. Foaming of the medium and a decline in cell density were observed at 0.6 and 0.7v v⁻¹ min⁻¹ (Fig. 6). The optimum nitrogen flow rate of 0.5v v⁻¹ min⁻¹ gave a biomass yield of 4.0g wet weight per litre of culture eluate, giving the bioreactor a productivity of 0.8g per hour per litre culture volume.

### Biomass Production

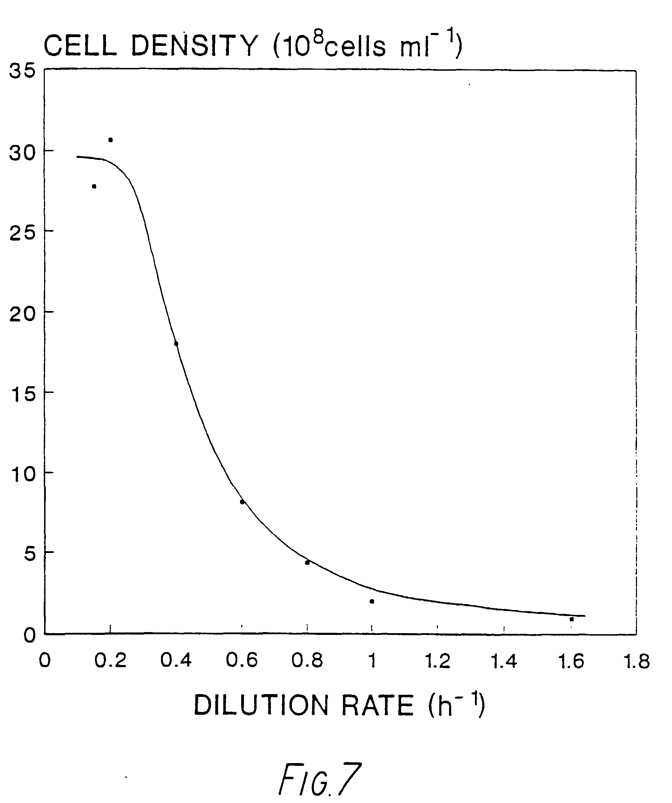

An optimal doubling time at 90°C of approximately 50 minutes had been previoulsy reported for *Pryococcus furiosus* (Fiala and Stetter 1986). It was considered, therefore, that the cell densities achieved might be maintainable at dilution rates higher than 0.2h⁻¹, giving an increase in the biomass production rate of the bioreactor. Dilution rates from 0.2 -1.6h⁻¹ were tested, however, progressively lower equilibrium cell densities were achieved as the dilution rate was increased (Fig. 7).

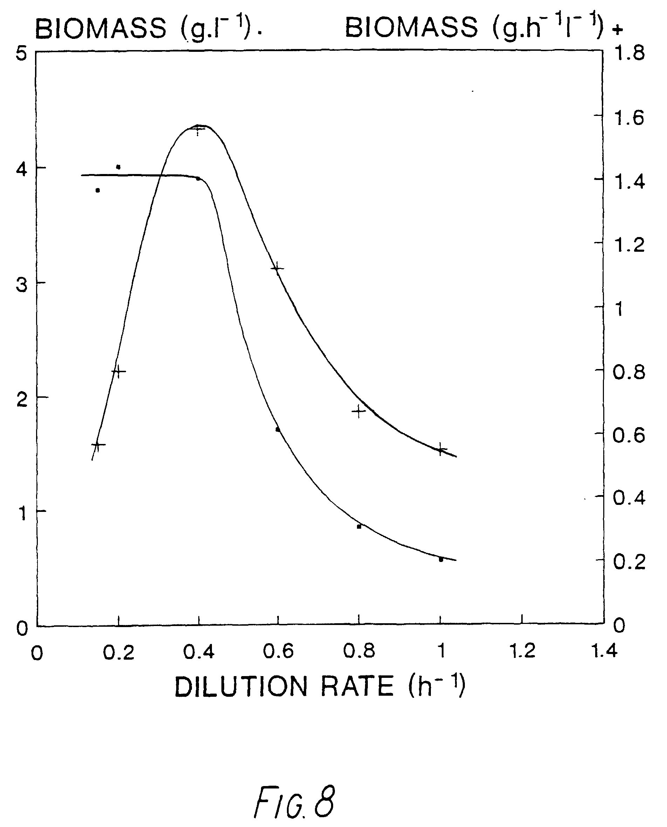

Biomass per litre of culture eluate also declined with increasing dilution rate, but the rate of decline was lower (Fig. 8). This correlated well with an apparent increase in cell size observed under the microscope. Total biomass productivity of the bioreactor (biomass per litre of culture eluate x dilution rate) was highest at a dilution rate of 0.4h⁻¹, generating 1.56g wet weight of cells per hour per litre culture volume (Fig.8).

### Example 2

*Pyrococcus furiosus* was grown at a cell density of 10¹⁰ml⁻¹ (4g l⁻¹ wet weight of cells) when the medium was modified with the peptone (5g l⁻¹) being replaced by maltose (20mM) and an amino acid cocktail formulated as follows: Glutamate and Glycine, 80mg l⁻¹; Arginine, 60mg l⁻¹; Cysteine and Proline, 50mg l⁻¹; Threonine, Histidine, Isoleucine, Lysine, Leucine and Asparagine, 40mg l⁻¹; Methionine, Phenylalanine, Serine, Alanine, Tryptophan, 30mg l⁻¹ and Valine, Glutamine and Aspartate, 20mg l⁻¹. All other conditions were as in Example 1.

### Example 3

*Pyrococcus furiosus* was grown at a cell density of 6.6 x 10⁹ ml⁻¹ (biomass 3.5gL⁻¹) under the conditions of Example 2 when the yeast extract (1g L⁻¹) was omitted.

This represents high cell density growth on a defined medium and compares with 2 x 10⁷ml⁻¹ obtained in the only other reports of growth of *Pyroccus furiosus* on a defined medium (Blumentals et al, 1990 and Snowden et al, 1992)

### Example 4

Using a gas lift fermenter steady state cell densities of 1 x 10⁹ ml⁻¹ (at 95°C, pH 6.0, dilution rate 0.1h⁻¹ and nitrogen flow rate of 0.2 v v⁻¹ min⁻¹) were obtained for *Pyrobaculum islandicum.* The growth medium in this case was modified from the basal mineral medium of Allen, 1959 and contained:-

| | |
|---|---|
| Bacto Peptone | 0.5g l⁻¹ |
| Yeast Extract | 0.2g l⁻¹ |
| 1- cysteine | 0.5g l⁻¹ |
| Sodium thiosulphate 5H₂O | 2g l⁻¹ |
| Sodium tungstate | 3.3mg l⁻¹ |
| Boric acid | 7.5mg l⁻¹ |
| Sodium molybdate 2H₂O | 0.15mg l⁻¹ |
| Sodium selenite 5H₂O | 5µg l⁻¹ |

in addition to the mineral salts of Allen. The pH was adjusted to 6.0 with HCl.

### REFERENCES

1. Allen M.B., (1959). Studies with *Cyanidium caldanium* an anomalously pigmented chlorophyte. Arch. Microbiol 32:270-277.
2. Balch W.E., Fox G.E., Magrum L.I., Woese C.R., and Wolfe R.S., (1979). Methariogens: reevaluation of a unique biological group. Microbiol. Rev. 43:260-296.
3. Blumentals I.I., Brown S.H., Schico R.N., Skaja A.K., Costantino H.R. and Kelly R.M. (1990). The hyperthermophilic archaebacterium *Pyrococcus furiosus.* Development of culturing protocols, perspectives on scale up and potential applications. Am. N.Y. Acad.Sci 589:301-314.
4. Bryant F.0., Adams M.W.W (1989). Characterization of hydrogenase from the hyperthermophilic archaebacterium *Pyrococcus furiosus.* J Biol Chem 264:5070-5079.
5. Fiala G., Stetter K.0. (1986). *Pyrococcus furiosus* sp. nov. represents a novel genus of marine heterotrophic archaebacteria growing optimally at 100°C. Arch Microbiol 145:55-61
6. Huber R., KristJannson J.K. and Stetter K.O. (1987), *Pyrobaculum* gen.nov., a new genus of neutrophilic, rod-shaped archaebacteria from continental solfataras growing optimally at 100°C. Arch. Microbiol 149:95-101.
7. Parameswaran A.K., Su W-W., Schicho R.N., Provan C.N., Malik B. and Kelly R.M. (1988). Engineering Considerations for growth of bacteria at temperatures around 100°C. Appl. Biochem. Biotechnol. 18:53-73.
8. Snowden L.J., Blumentals I.I. and Kelly R.M. (1992). Regulation of proteolytic activity in the hyperthermophile *Pyrococcus furiosus.* Appl. Environ. Microbiol. 58:1134-1141.
9. Stetter K.O., Konig H., Stackebrandt E. (1983). *Pyrodictium* gen. nov., a new genus of submarine disc-shape sulphur reducing archaebacteria growing optimally at 105°C, System Appl. Microbiol 4:535-551.

## Claims

1. A process for producing thermophilic archaea in medium essentially free of elemental sulphur and wherein an inert gas is continuously introduced into the medium so as to remove hydrogen and/or hydrogen sulphide evolved by the archaea characterised in that said archaea are grown continuously in the presence of a nutrient medium containing assimilable sources of carbon and nitrogen and any necessary complex nutrients required for cell growth, and in that the inert gas is continuously introduced into the culture medium at a rate of between 0.1 and 1 vv⁻¹min⁻¹.

2. A process according to Claim 1, characterised in that the inert gas is continuously introduced into the culture medium at a rate of between 0.20 and 0.75 vv⁻¹min⁻¹.

3. A process according to any preceding claim, characterised in that the archaea are grown in a gas-lift fermenter.

4. A process according to any preceding claim, characterised in that the inert gas is selected from nitrogen, argon, helium, and sulphur hexafluoride.

5. A process according to any preceding claim, characterised in that the concentration of ionised salts in the medium is maintained at between 0.1 and 1M.

6. A process according to any preceding claim, characterised by being carried out at a dilution rate of ≤ 0.4 h⁻¹.

7. A process according to any preceding claim, characterised in that said archaea are of the genus *Pyrococcus.*

8. A process according to any of Claims 1 to 6, characterised in that said archaea are of the genus *Pyrobaculum.*

## Patentansprüche

1. Verfahren zur Herstellung thermophiler Archaebakterien in einem Medium, das im wesentlichen frei von elementarem Schwefel ist, und bei dem ein Inertgas kontinuierlich in das Medium zur Entfernung von Wasserstoff und/oder Wasserstoffsulfiden, die durch die Archaebakterien entwickelt werden, eingeleitet wird, dadurch gekennzeichnet, daß die Archaebakterien kontinuierlich in Anwesenheit eines Nährmediums gezogen werden, das assimilierbare Quellen von Kohlenstoff und Stickstoff und notwendige komplexe Nährstoffe enthält, die für das Zellwachstum erforderlich sind, und das Inertgas kontinuierlich in das Kulturmedium mit einer Geschwindigkeit zwischen 0,1 und 1 VV⁻¹ min⁻¹ eingeleitet wird.

2. Verfahren nach Anspruch 1, dadurch gekennzeichnet, daß das Inertgas in das Kulturmedium kontinuierlich mit einer Geschwindigkeit zwischen 0,20 und 0,75 VV⁻¹ min⁻¹ eingeleitet wird.

3. Verfahren nach einem der vorstehenden Ansprüche, dadurch gekennzeichnet, daß die Archaebakterien in einem Gaslift-Fermenter gezogen werden.

4. Verfahren nach einem der vorstehenden Ansprüche, dadurch gekennzeichnet, daß das Inertgas aus Stickstoff, Argon, Helium und Schwefelhexafluorid ausgewählt wird.

5. Verfahren nach einem der vorstehenden Ansprüche, dadurch gekennzeichnet, daß die Konzentration ionisierter Salze im Medium zwischen 0,1 und 1 M gehalten wird.

6. Verfahren nach einem der vorstehenden Ansprüche, dadurch gekennzeichnet, daß es bei einer Verdünnungsrate von ≤ 0,4 h⁻¹ durchgeführt wird.

7. Verfahren nach einem der vorstehenden Ansprüche, dadurch gekennzeichnet, daß die Archaebakterien der Gattung Pyrococcus angehören.

8. Verfahren nach einem der Ansprüche 1 bis 6, dadurch gekennzeichnet, daß die Archaebakterien der Gattung Pyrobaculum angehören.

## Revendications

1. Procédé de production d'archaebactéries thermophiles dans un milieu essentiellement exempt de soufre élémentaire et dans lequel on introduit en continu un gaz inerte dans le milieu de manière à éliminer l'hydrogène et/ou le sulfure d'hydrogène dégagés par les archaebactéries, caractérisé en ce que l'on cultive en continu lesdites archaebactéries en présence d'un milieu nutritif contenant des sources assimilables de carbone et d'azote et tous les agents nutritifs complexes nécessaires à la croissance des cellules, et en ce que l'on introduit en continu le gaz inerte dans le milieu de culture à une vitesse comprise entre 0,1 et 1 vv⁻¹mn⁻¹.

2. Procédé selon la revendication 1, caractérisé en ce que l'on introduit en continu le gaz inerte dans le milieu de culture à une vitesse comprise entre 0,20 et 0,75 vv⁻¹mn⁻¹.

3. Procédé selon l'une quelconque des revendications précédentes, caractérisé en ce que l'on cultive les archaebactéries dans un fermenteur à agitation par un gaz.

4. Procédé selon l'une quelconque des revendications précédentes, caractérisé en ce que le gaz inerte est choisi parmi l'azote, l'argon, l'hélium et l'hexafluorure de soufre.

5. Procédé selon l'une quelconque des revendications précédentes, caractérisé en ce que l'on maintient la concentration des sels ionisés dans le milieu entre 0,1 et 1M.

6. Procédé selon l'une quelconque des revendications précédentes, caractérisé en ce qu'il s'effectue à une vitesse de dilution inférieure ou égale à 0,4 h⁻¹.

7. Procédé selon l'une quelconque des revendications précédentes, caractérisé en ce que lesdites archaebactéries sont du genre *Pyrococcus.*

8. Procédé selon l'une quelconque des revendications 1 à 6, caractérisé en ce que lesdites archaebactéries sont du genre *Pyrobaculum.*
